# EUROPEAN PATENT APPLICATION

(11) **EP 4 349 966 A1**
(43) Date of publication of application: **10.04.2024**
(21) Application number: 23789477.9
(22) Date of filing: 11.07.2023
(51) Int. Cl.: C12N 5/071

(54) **METHOD FOR CONSTRUCTING ALVEOLAR ORGANOID USING INDUCED PLURIPOTENT STEM CELLS DERIVED FROM ALVEOLAR CELLS**

(30) Priority: 26.08.2022 KR 20220107869
(71) Applicant: The Catholic University Of Korea Industry-Academic Cooperation Foundation, Seocho-gu Seoul 06591 (KR)
(72) Inventor: KIM, Sung Won, Seoul 06001 (KR); JO, Ayoung, Seoul 07075 (KR); LIM, Min Jae, Pyeongtaek-si Gyeonggi-do 17880 (KR)
(74) Representative: Dehns
(86) International application number: PCT/KR2023/009885
(87) International publication number: WO 2024/043521

(57) **Abstract**

The present invention relates to a method of manufacturing an alveolar organoid using alveolar cell-derived induced pluripotent stem cells. The alveolar organoid manufactured according to the present invention is differentiated from the human alveolar cell-derived induced pluripotent stem cells, and thus induced pluripotent stem cells may efficiently differentiate into alveolar organoids due to the epigenetic memory of alveolar cells. Therefore, the alveolar organoids are expected to be effectively used for research on the pathological process of respiratory diseases, screening for finding therapeutic drugs, and the like.

## Description

### [Technical Field]

The present invention relates to a method of manufacturing an alveolar organoid using human alveolar cell-derived induced pluripotent stem cells.

This application claims priority to and the benefit of Korean Patent Application No. filed on August 26, 2022, and all the contents disclosed in the specification and drawings of the application are incorporated in this application.

### [Background Art]

Organoids are mini-organs made by culturing or recombining cells isolated from stem cells or organ cells, and are characterized by having a structure and function very similar to that of the corresponding organ, allowing differentiation as close as possible to actual tissue.

In recent years, research on organoids as regenerative medicine or cell therapy is actively underway. Attempts are mainly made in animal experiments to investigate actual drug metabolism reactions. However, because the genetic structures of humans and animals are not 100% identical, substances that have no reaction in animals may be fatal to humans, and the problem of violating animal rights has been raised. Also, because the two-dimensionally cultured cells are immortalized cells and differ from actual organs in terms of the degree of drug metabolism, it is difficult to perform accurate experiments. In addition, the best way to evaluate drug metabolism is to directly use human organ cells, but there are problems of limitations in obtaining the human tissues, and it is difficult to obtain a large amount of human tissue due to difficult *ex vivo* proliferation.

Meanwhile, research results have recently announced that an increase in the concentration of fine dust, which is an air pollutant, is related to an increase in the risk and mortality related to lung diseases including cancer. Also, lung diseases are known as the third leading cause of death in the world, but the principle of restoring damaged lungs is not known.

Despite the fact that the lungs are structurally more complex than other organs and have a variety of cell types, much research has not yet been conducted on the lungs. In particular, alveolar type 2 cells (AT2 cells) in the lung tissue account for approximately 7% of alveolar epithelial cells (The Formation of Pulmonary Alveoli; Stephen E. McGowan; in The Lung (Second Edition), 2014), and play a role in preventing collapse by producing surfactant proteins to maintain lung function and surface tension.

However, when the alveolar type 2 cells are cultured in a 2D manner, the alveolar type 2 cells have the same phenotype as alveolar type 1 cells, making it difficult to culture alveolar type 2 cells, and it is difficult to maintain the alveolar type 2 cells in human lung tissue.

### [Disclosure]

### [Technical Problem]

Therefore, the present inventors have made efforts to manufacture an alveolar organoid using human alveolar cell-derived induced pluripotent stem cells, and developed a medium suitable for the manufacture of alveolar organoids.

Accordingly, it is an aspect of the present invention to provide a method of manufacturing an alveolar organoid from alveolar cell-derived induced pluripotent stem cells.

It is another aspect of the present invention to provide an alveolar organoid manufactured using the method of manufacturing an alveolar organoid according to the present invention.

It is still another aspect of the present invention to provide a kit for manufacturing an alveolar organoid.

It is yet another aspect of the present invention to provide a composition for manufacturing an alveolar organoid.

It is yet another aspect of the present invention to provide a tissue therapeutic agent including the alveolar organoid according to the present invention.

It is yet another aspect of the present invention to provide a method of screening therapeutic agents for lung-related diseases using the alveolar organoid according to the present invention.

However, the technical objects of the present invention are not limited to the technical object as described above, and other objects of the present invention which are not stated herein will become more apparent to those of ordinary skill in the art by describing exemplary embodiments thereof in detail.

### [Technical Solution]

To solve the above problems, according to an aspect of the present invention, there is provided a method of manufacturing an alveolar organoid from alveolar cell-derived induced pluripotent stem cells, which includes the following steps:
a) culturing alveolar cell-derived induced pluripotent stem cells in a medium for inducing endoderm cell differentiation to induce the differentiation of the induced pluripotent stem cells into endoderm cells;
b) inducing the differentiation of the endoderm cells of step a) into anterior foregut endoderm cells in an anterior foregut endoderm medium (AFM);
c) inducing the differentiation of the anterior foregut endoderm cells of step b) into an alveolar progenitor in a progenitor differentiation medium; and
d) culturing the alveolar progenitor of step c) in an alveolar medium (AM).

According to one exemplary embodiment of the present invention, the alveolar cells may be alveolar type 2 cells, but the present invention is not limited thereto.

According to another exemplary embodiment of the present invention, the AFM may include one or more selected from Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, antibiotic-antimycotic, ascorbic acid, Glutamax, recombinant human BMP-4, and CHIR99021, but the present invention is not limited thereto.

According to still another exemplary embodiment of the present invention, the AM may include one or more selected from the group consisting of Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, and antibiotic-antimycotic, but the present invention is not limited thereto.

According to yet another exemplary embodiment of the present invention, steps b) to d) may be performed by a three-dimensional (3D) culture method, but the present invention is not limited thereto.

According to another aspect of the present invention, there is provided a kit for manufacturing an alveolar organoid, which includes a medium for inducing endoderm cell differentiation, an anterior foregut endoderm medium (AFM), a progenitor differentiation medium, and an alveolar medium (AM).

According to one exemplary embodiment of the present invention, the kit may further include instructions describing the method of manufacturing an alveolar organoid from the alveolar cell-derived induced pluripotent stem cells according to the present invention, but the present invention is not limited thereto.

According to still another aspect of the present invention, there is provided a composition for manufacturing an alveolar organoid.

According to yet another aspect of the present invention, there is provided an alveolar organoid manufactured using the method of manufacturing an alveolar organoid according to the present invention.

According to yet another aspect of the present invention, there is provided a tissue therapeutic agent including the alveolar organoid according to the present invention.

According to yet another aspect of the present invention, there is provided a method of preventing or treating a lung-related disease, which includes: administering and/or transplanting the alveolar organoid according to the present invention to a subject in need thereof.

According to yet another aspect of the present invention, there is provided a use of the alveolar organoid according to the present invention for the prevention or treatment of a lung-related disease.

According to yet another aspect of the present invention, there is provided a use of the alveolar organoid according to the present invention for the manufacture of a therapeutic agent for lung-related diseases.

According to yet another aspect of the present invention, there is provided a method of screening a therapeutic agent for lung-related diseases using the alveolar organoid according to the present invention.

According to one exemplary embodiment of the present invention, the method includes: treating the organoid with a test substance; and selecting the test substance as a therapeutic agent for a lung-related disease when the expression of a biomarker protein of a lung-related disease or mRNA encoding the same increases or decreases after the treatment with the test substance compared to before the treatment, but the present invention is not limited thereto.

According to another exemplary embodiment of the present invention, the lung-related disease may be selected from the group consisting of cystic fibrosis, respiratory distress syndrome, acute respiratory distress syndrome, pulmonary tuberculosis, coughing, bronchial asthma, coughing due to increased airway hyperresponsiveness, flu syndrome, cough suppression, airway hyperresponsiveness, tuberculosis , asthma, chronic obstructive pulmonary disease, pulmonary emphysema, pulmonary fibrosis, idiopathic pulmonary fibrosis, reversible airway obstruction, adult respiratory disease complex, pigeon breeder's disease, farmer's lung, bronchopulmonary dysplasia, airway diseases, pulmonary emphysema, allergic bronchopulmonary aspergillosis, allergic bronchitis, bronchiectasis, occupational asthma, reactive airway disease syndrome, interstitial lung disease, and parasitic lung disease, but the present invention is not limited thereto.

### [Advantageous Effects]

An alveolar organoid manufactured according to the present invention is differentiated from human alveolar cell-derived induced pluripotent stem cells, and thus induced pluripotent stem cells can repeatedly and efficiently differentiate into alveolar organoids and actually reproduced in a similar manner to alveoli due to the epigenetic memory of alveolar cells. Therefore, the alveolar organoids are expected to be effectively used for research on the pathological process of respiratory diseases, screening for finding therapeutic drugs, and the like.

### [Description of Drawings]

FIG. 1 is a diagram showing a process of reprogramming human alveolar cells (AT2 cells) to manufacture induced pluripotent stem cells (iPSCs).
FIG. 2 shows the results of analyzing the protein characteristics of the induced pluripotent stem cells (iPSCs) manufactured by reprogramming human alveolar cells (AT2 cells) through an immunofluorescence staining method. Here, the left diagram of FIG. 2 shows relative expression levels of undifferentiated iPSC markers Oct3/4, NANOG, and LIN28 in the AT2 cells, and the right diagram of FIG. 2 shows the results of observing the expression of Oct3/4, SSEA4, TRA-1-60, and TRA-1-81 using a confocal microscope.

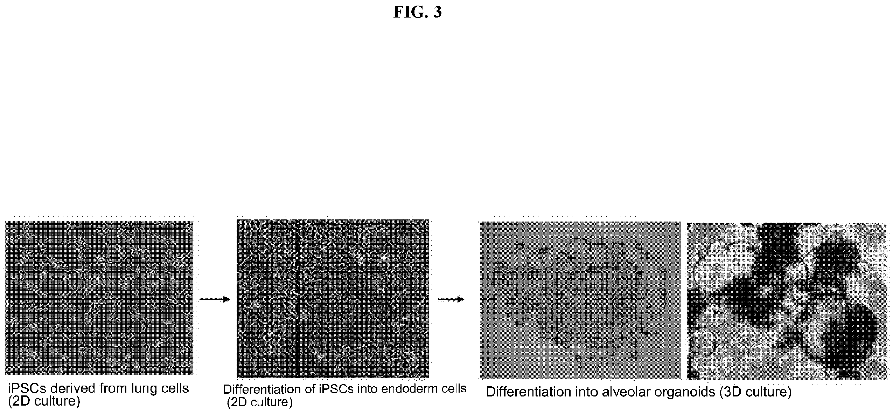

FIG. 3 is a diagram showing the results of observing the course of differentiation of alveolar organoids from the induced pluripotent stem cells (iPSC) manufactured by reprogramming human alveolar cells (AT2 cells).
FIG. 4 is a diagram showing a schematic protocol for the differentiation of alveolar organoids from the induced pluripotent stem cells (iPSC) manufactured by reprogramming human alveolar cells (AT2 cells).
FIG. 5 is a diagram showing the results of confirming, through an immunofluorescence staining method, that the induced pluripotent stem cells (iPSCs) manufactured by reprogramming human alveolar cells (AT2 cells) are differentiated into endoderm, which shows that the induced pluripotent stem cells (iPSCs) are normally differentiated into endoderm using endoderm-specific factors SOX17 and FOXA2.
FIG. 6 is a diagram showing the results of observing the course of differentiation of alveolar organoids from the induced pluripotent stem cells (iPSC) manufactured by reprogramming human alveolar cells (AT2 cells) through an immunofluorescence staining method.

### [Best Mode]

The present invention provides a method of manufacturing an alveolar organoid from alveolar cell-derived induced pluripotent stem cells, which includes the following steps:
a) culturing alveolar cell-derived induced pluripotent stem cells in a medium for inducing endoderm cell differentiation to induce the differentiation of the induced pluripotent stem cells into endoderm cells;
b) inducing the differentiation of the endoderm cells of step a) into anterior foregut endoderm cells in an anterior foregut endoderm medium (AFM);
c) inducing the differentiation of the anterior foregut endoderm cells of step b) into an alveolar progenitor in a progenitor differentiation medium; and
d) culturing the alveolar progenitor of step c) in an alveolar medium (AM).

In the present invention, the term "organoid" refers to a 3D assembly of one or more cell types that mimic the superficial appearance or actual structure or function of a tissue or organ.

According to one embodiment of the present invention, the alveolar cells may be alveolar type 2 cells (AT2 cells), but the present invention is not limited thereto.

The alveolar type 2 cells (AT2 cells) may be isolated from lung tissue, subcultured after isolation, or commercially available, but the present invention is not limited thereto.

In the present invention, the term "lung tissue" may include all lung tissue structures and associated tissues, including, but not limited to, veins, arteries, vessels, capillaries, and cells of the type that are part of, or associated with, such structures; lung and pleural tissues; and vascular smooth muscle, pericytes, and vascular endothelial lineages and/or phenotypes, but the present invention is not limited thereto. The lung tissue may be isolated from mammals, such as humans, mice, rats, guinea pigs, rabbits, monkeys, pigs, horses, cattle, sheep, antelopes, dogs, or cats, but the present invention is not limited thereto. Preferably, the mammal may be a human. Such lung tissue may be obtained by a method known in the art.

In the present invention, the term "differentiation" means a phenomenon in which specialized structures and functions are developed while cells proliferate and grow, that is, cells, tissues, and the like change in form or function to perform a given task.

As used in the present invention, the term "induced pluripotent stem cells (iPSCs)" refers to cells induced by artificially performing a dedifferentiation process (reprogramming) on already completely differentiated adult cells, and have pluripotency. The induced pluripotent stem cells can differentiate into cells of various organs such as the brain, heart, and the like. In the present invention, the induced pluripotent stem cells may be cells obtained by dedifferentiation of human alveolar type 2 cells. However, the type of tissue is not particularly limited as long as it is derived from humans.

According to one embodiment of the present invention, the alveolar cell-derived induced pluripotent stem cells are characterized by being manufactured by introducing a virus including a dedifferentiation inducer into alveolar cells. More specifically, the alveolar cell-derived induced pluripotent stem cells may be alveolar cells, particularly alveolar type 2 cells, which are manufactured using a method of manufacturing induced pluripotent stem cells, which includes the following steps, but the present invention is not limited thereto:
(S1) isolating alveolar cells;
(S2) introducing a virus including a dedifferentiation inducer into the alveolar cells; and
(S3) inducing the dedifferentiation of the virus-introduced alveolar cells into induced pluripotent stem cells.

As used in the present invention, the term "dedifferentiation" refers to an epigenetic reversal process that enables the formation of new differentiated tissue by returning partially or finally differentiated cells to an undifferentiated state such as a pluripotent or multipotent state. This dedifferentiation phenomenon is possible because it is a reversible process in which the epigenetic changes of the cell genome are not imprinted but may be erased and reset. Dedifferentiation is also referred to as "reprogramming," and refers to a process of changing the genetic and phenotypic profile of partially or finally differentiated cells to resemble those of embryonic stem cells. For example, the changes include a change in a methylation pattern, a change in an expression level of stem cell genes, and the like. An artificial dedifferentiation process includes a dedifferentiation process performed using a virus-mediated or non-viral vector using a lentivirus belonging to the family *Retroviridae, Sendai* virus belonging to the family Paramyxoviridae, and the like or performed by introducing a non-viral mediated dedifferentiation factor using proteins, cell extracts, and the like, and a dedifferentiation process using a stem cell extract, a compound, and the like. In this case, it is preferable to use the *Sendai* virus in the present invention.

The "dedifferentiation inducer (a reprogramming-inducing gene)" includes genes capable of reprogramming completely differentiated cells. In particular, Oct-4, Sox2, Klf4, and c-Myc are called Yamanaka factors.

In the present invention, the virus of step (S2) may be a *Sendai* virus, and the dedifferentiation inducer of step (S2) may include any one or more selected from the group consisting of Klf4, c-Myc, and polycistronic Klf4-Oct3/4-Sox2. According to one embodiment of the present invention, Klf4, c-Myc, and polycistronic Klf4-Oct3/4-Sox2 (Cytotune^{™} 2.0 *Sendai* mix) may be used as the *Sendai* virus dedifferentiation inducer, but the present invention is not limited thereto. In this case, dedifferentiation inducers may be used without limitation as long as they can induce the dedifferentiation of alveolar cells into induced pluripotent stem cells.

c-Myc is an oncogene that performs various intracellular functions such as cell growth, differentiation, proliferation, apoptosis, and transformation into cancer cells. It was also found that c-Myc is a hypostatic gene for leukemia inhibitory factor (LIF)/STAT3 and Wnt signaling mechanisms, which are major mechanisms for maintaining pluripotency (Sears et al., Genes Dev, 14:2501-2514, 2000). A c-Myc transcription factor is expected to play a role in preventing the inhibition of cell proliferation in dedifferentiated pluripotent stem cells. Also, c-Myc not only binds to Myc recognition sites present on the genome, but also functions to help Oct-4 and Sox2 bind to a target gene by changing the chromatin structure (Lewitzky and Yamanaka, Current Opinion in Biotechnology, 18:467-473, 2007).

Klf4 is involved in growth inhibition to play a role in regulating the cell cycle. According to recent studies, it was found that, similar to c-Myc, Klf4 acts as a hypostatic gene for STAT3, and inhibits the differentiation of mouse embryonic stem cells by maintaining Oct-4 expression when overexpressed (Li et al., Blood, 105:635-637, 2005).

In the present invention, in order to form pluripotent stem cells through reprogramming, the delivery of a dedifferentiation inducer into alveolar cells needs to be performed. Specifically, genes encoding dedifferentiation inducers of polycistronic Klf4-Oct3/4-Sox2, c-Myc, and Klf4 may be delivered into alveolar cells using *Sendai* virus as a transporter, but the combination of dedifferentiation factors to be delivered according to the type of transporter is not limited thereto.

According to one embodiment of the present invention, the alveolar cell-derived induced pluripotent stem cells of step a) may be cultured in a cell culture dish coated with vitronectin before performing step a). When 60 to 90%, 60 to 80%, or 70 to 80% of the culture dish is filled with the alveolar cell-derived induced pluripotent stem cells, the alveolar cell-derived induced pluripotent stem cells may be cultured in a medium for inducing endoderm cell differentiation to induce the differentiation into endoderm cells

In the present invention, the "medium" refers to a medium capable of supporting the induction of differentiation into desired cells (including tissue or organoids) and survival *in vitro,* and includes all types of media commonly used in the art suitable for cell culture. The media and culture conditions may be selected according to the type of cells.

In the present invention, the "medium for inducing endoderm cell differentiation" refers to a culture medium that promotes the differentiation of induced pluripotent stem cells (iPSCs) into endoderm cells. According to one embodiment of the present invention, a STEMdiff^{™} Definitive Endoderm kit (STEMCELL #05110) was used, but all types of media may be used without limitation as long as they can induce the differentiation of iPSCs into lung endoderm cells.

The STEMdiff^{™} Definitive Endoderm kit may include Endoderm Basal Medium, Definitive Endoderm Supplement MR (100), and Definitive Endoderm Supplement CJ (100). Specifically, the STEMdiff^{™} Definitive Endoderm kit may include 100 mL of Endoderm Basal Medium, 0.35 mL of Definitive Endoderm Supplement MR (100), and 1.1 mL of Definitive Endoderm Supplement CJ (100).

According to one embodiment of the present invention, the culturing of the alveolar cell-derived induced pluripotent stem cells in the medium for inducing endoderm cell differentiation in step a) may include: adding MR supplement plus CJ supplement to the medium for inducing endoderm cell differentiation, culturing the alveolar cell-derived induced pluripotent stem cells for 2 days, adding the CJ supplement only, and culturing alveolar cell-derived induced pluripotent stem cells for 1 to 5 days, 1 to 3 days, 1 to 2 days, 2 to 4 days, or 2 to 3 days.

As used in the present invention, the term "endoderm" refers to the innermost germ layer among the three germ layers (ectoderm, mesoderm, and endoderm) formed by the migration of cells to the inside of the gastrula as a result of gastrulation. In this case, the endoderm differentiates into respiratory and digestive organs such as the lungs, liver, pancreas, and the like. Therefore, in the present invention, efficient differentiation into endoderm cells is required as a prerequisite in order to promote the differentiation of induced pluripotent stem cells into alveolar organoids (Tissue Engineering and Regenerative Medicine 2007, Vol. 4, No. 2, pp. 142-149).

According to one embodiment of the present invention, the endoderm cells may express endoderm-specific factors SOX17 and FOXA2, but the present invention is not limited thereto.

In the present invention, the anterior foregut endoderm medium (AFM) refers to a culture medium that promotes the differentiation of endoderm cells into anterior foregut endoderm cells, and may include one or more selected from the group consisting of Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, antibiotic-antimycotic, ascobic acid, Glutamax, recombinant human BMP-4, and CHIR99021, but the present invention is not limited thereto. Specifically, the AFM may include 50 to 150 µM IBMX, 0.5 to 3% B27 supplement, 0.1 to 2% N2 supplement, 20 to 70 nM dexamethasone, 0.1 to 2% BSA, 0.5 to 1.5 mM calcium chloride (CaCl₂), 10 to 20 mM HEPES, 10 to 200 µM 8-BrcAMP, 1 to 20 ng/mL recombinant human KGF, 1 to 20 ng/mL recombinant human FGF-10, 0.1 to 2% anti-antibiotic antimycotic, 20 to 100 µg/mL ascobic acid, 0.5 to 5 mM Glutamax, 1 to 20 ng/mL recombinant human BMP-4, and/or 1 to 10 µM CHIR99021, based on 500 mL of Ham's F12, but the present invention is not limited thereto.

The culturing period of step b) is not particularly limited, and the culturing may be, for example, performed for 1 to 8 days, 1 to 5 days, 1 to 3 days, 1 to 2 days, 2 to 5 days, 2 to 4 days, 2 to 3 days, 3 to 7 days, 3 to 4 days, 4 to 7 days, 4 to 5 days, 1 day, 2 days, 3 days, 4 days, 5 days, 6 days, or 7 days. According to one embodiment of the invention, the culturing may be performed for 7 days, but the present invention is not limited thereto.

As used in the present invention, the term "progenitor differentiation medium" refers to a culture medium that promotes the differentiation of anterior foregut endoderm cells into progenitor cells. According to one embodiment of the present invention, an SAGM medium may be used.

The "SAGM medium" or "small airway growth medium" refers to a growth medium including at least one of hydrocortisone, an epidermal growth factor, epinephrine, transferrin, insulin, retinoic acid, triiodothyronine, and fatty acid-free bovine serum albumin.

In the present invention, the term "progenitor" refers to a cell that is differentiated into a specific type of cell or differentiated to form a specific type of tissue. In this case, the progenitor is partially differentiated from stem cells, and the stem cells may finally differentiate into adult cells through a progenitor step.

In the present invention, the culturing period of step c) is not particularly limited, and the culturing may be, for example, performed for 1 to 8 days, 1 to 5 days, 1 to 3 days, 1 to 2 days, 2 to 5 days, 2 to 4 days, 2 to 3 days, 3 to 7 days, 3 to 4 days, 4 to 7 days, 4 to 5 days, 1 day, 2 days, 3 days, 4 days, 5 days, 6 days, or 7 days. According to one embodiment of the present invention, the culturing may be performed for 7 days, but the present invention is not limited thereto.

According to one embodiment of the present invention, the alveolar progenitor may be one in which an epithelial cell factor, EPCAM, is expressed.

In the present invention, the culturing period of step d) is not particularly limited, but the alveolar progenitor may be preferably cultured for up to 90 days. When the cell density becomes too high during the culturing period, it may further include: repeating the following steps: isolating cells using Accutase and embedding the isolated cells, but the present invention is not limited thereto.

As used in the present invention, the term "alveolar medium (AM)" refers to a culture medium that promotes the differentiation of precursor cells into final adult cells. In the present invention, the AM may include one or more selected from the group consisting of Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, and antibiotic-antimycotic, but the present invention is not limited thereto. Specifically, the AM may include 50 to 150 µM IBMX, 0.5 to 3% B27 supplement, 0.1 to 2% N2 supplement, 20 to 70 nM dexamethasone, 0.1 to 2% BSA, 0.5 to 1.5 mM calcium chloride (CaCl₂), 10 to 20 mM HEPES, 10 to 200 µM 8-BrcAMP, 1 to 20 ng/mL recombinant human KGF, 1 to 20 ng/mL recombinant human FGF-10, and/or 0.1 to 2% antibiotic-antimycotic, based on 500 mL of Ham's F12, but the present invention is not limited thereto.

According to one embodiment of the present invention, step a) may be performed in a 2D culture vessel, and steps b) to d) may be performed in a 3D culture vessel, but the present invention is not limited thereto. In this case, each step may be performed in a culture vessel suitable for cell differentiation at each step.

In the present invention, steps b) to d) may be performed by a 3D culture method in which the cells are cultured in a 3D culture vessel. Here, the specific conditions for 3D culturing are not particularly limited, and the culturing may be performed by a method commonly performed in the art. For example, the culturing may be performed using an adhesive material selected from the group consisting of polyester, polyalkylene, polyfluorochloroethylene, polyvinyl chloride, polystyrene, polysulfone, cellulose acetate, glass fibers, ceramic particles, Matrigel, extracellular matrix components (e.g., fibronectin, chondronectin, laminin), collagen, poly-L lactic acid, and inert metal fibers. Preferably, the culturing may be performed using Matrigel.

According to one embodiment of the present invention, the endoderm cells of step a) express endoderm-specific factors SOX17 and FOXA2, and the alveolar progenitor of step c) expresses EPCAM, which is an epithelial cell factor. Here, because the alveolar progenitor cultured in the AM in step d) and differentiated into alveolar organoids shows the expression of alveolar type 1 and alveolar type 2 cells, the alveolar organoids may be efficiently manufactured in the present invention by confirming each of processes for differentiating induced pluripotent stem cells into alveolar organoids step by step.

According to another aspect of the present invention, the present invention provides a kit for manufacturing an alveolar organoid, which includes a medium for inducing endoderm cell differentiation, an anterior foregut endoderm medium (AFM), a progenitor differentiation medium, and an alveolar medium (AM).

In the present invention, the kit may further include instructions describing a method for manufacturing an alveolar organoid from alveolar cell-derived induced pluripotent stem cells according to the present invention, but the present invention is not limited thereto.

The instructions may be attached to a vessel, or may be packaged independently of the vessel.

Also, in addition to the medium included in the kit, any constituents known in the art to be necessary for differentiating alveolar cell-derived induced pluripotent stem cells into alveolar organoids may be included without limitation.

In addition, the kit may further include a culture dish, and the culture dish may be a culture dish for suspension culture or a culture dish for adherent culture.

The culture dish for adherent culture may be coated with a polypeptide, and the polypeptide may be, for example, vitronectin (VTN), laminin, fibronectin, polyornithine, or Matrigel.

According to still another aspect of the present invention, the present invention provides a composition for manufacturing an alveolar organoid.

The composition for manufacturing an alveolar organoid may have the same compositions as the AFM or AM, and may include one or more selected from the group consisting of Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, antibiotic-antimycotic, ascobic acid, Glutamax, recombinant human BMP4, and CHIR99021, but the present invention is not limited thereto.

In the present invention, the composition may further include a small airway growth medium (SAGM), but the present invention is not limited thereto.

The term "organoid manufacturing" used in the present invention includes all types of actins capable of generating or maintaining organoids. For example, it may include differentiating cells or cells isolated from a specific tissue into tissue or organ cells having a specific function, and/or surviving, growing, or proliferating organoids.

According to yet another aspect of the present invention, the present invention provides an alveolar organoid manufactured by the method according to the present invention. In the present invention, because the alveolar organoid is manufactured using alveolar cell-derived induced pluripotent stem cells, efficient differentiation into alveolar organoids may occur due to the epigenetic memory of the alveolar cells.

According to one embodiment of the present invention, the alveolar organoid may contain both alveolar type 2 cell and alveolar type 1 cells, but the present invention is not limited thereto.

According to yet another aspect of the present invention, the present invention provides a tissue therapeutic agent including the alveolar organoid, and the tissue may be lung tissue, but the present invention is not limited thereto.

According to yet another aspect of the present invention, the present invention provides a method for preventing or treating a lung-related disease, which includes: administering and/or transplanting the alveolar organoid to a subject in need thereof.

Also, the present invention provides a use of the alveolar organoid according to the present invention for the prevention or treatment of a lung-related disease.

In addition, the present invention provides a use of the alveolar organoid according to the present invention for the manufacture of a therapeutic agent for lung-related diseases.

In the present invention, the "subject" may be a human or non-human mammal that has developed a lung-related disease or is at risk of developing a lung-related disease. For example, the non-human mammal includes livestock and pets, such as sheep, cattle, pigs, dogs, cats, and mammals such as murines. Preferably, the subject is a human.

Non-limiting examples of the "lung-related disease" may include cystic fibrosis, respiratory distress syndrome, acute respiratory distress syndrome, pulmonary tuberculosis, coughing, bronchial asthma, coughing due to increased airway hyperresponsiveness, flu syndrome, cough suppression, airway hyperresponsiveness, tuberculosis, asthma, chronic obstructive pulmonary disease, pulmonary emphysema, pulmonary fibrosis, idiopathic pulmonary fibrosis, reversible airway obstruction, adult respiratory disease complex, pigeon breeder's disease, farmer's lung, bronchopulmonary dysplasia, airway diseases, pulmonary emphysema, allergic bronchopulmonary aspergillosis, allergic bronchitis bronchiectasis, occupational asthma, reactive airway disease syndrome, interstitial lung disease, parasitic lung disease, and the like.

In the present invention, the term "administration" refers to any action of introducing a pharmaceutical composition of the present invention to a patient using any suitable method. In this case, the composition of the present invention may be administered through various oral or parenteral administration routes as long as it can reach the target tissue.

In the present invention, the term "prevention" refers to any action of delaying a lung-related disease by administration/transplantation of the tissue therapeutic agent or alveolar organoid according to the present invention, the term "treatment" refers to any action of improving or beneficially changing the symptoms of a lung-related disease by administration/transplantation of the tissue therapeutic agent or alveolar organoid according to the present invention, and the term "improvement" refers to any action of reducing parameters related to a lung-related disease, for example, the severity of symptoms thereof, by administration/transplantation of the tissue therapeutic agent or alveolar organoid according to the present invention.

The tissue therapeutic agent of the present invention may be a pharmaceutical composition including the alveolar organoid as an active ingredient, and the pharmaceutical composition may further include appropriate carriers, excipients, and diluents commonly used in the preparation of the pharmaceutical composition.

In the present invention, the term "carrier" is also referred to as a vehicle, and refers to a compound that facilitates the addition of proteins or peptides into cells or tissues. For example, dimethyl sulfoxide (DMSO) is a carrier commonly used to facilitate the introduction of many organic substances into cells or tissue of organisms.

In the present invention, the term "diluent" is defined as a compound diluted in water that not only stabilizes the biologically active form of a target protein or peptide, but also dissolves the protein or peptide. Salts dissolved in a buffer solution are used as the diluent in the art. A commonly used buffer solution is phosphate buffered saline because it mimics the salt state of human body fluid. Because buffer salts can control the pH of a solution at low concentrations, buffer diluents may rarely modify the biological activity of a compound.

Also, the pharmaceutical composition according to the present invention may be formulated and used in the form of external preparations and sterile injection solutions such as powders, granules, tablets, capsules, suspensions, emulsions, syrups, aerosols, and the like using conventional methods. Carriers, excipients and diluents that may be included in the composition include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils. When formulated, the composition may be prepared using a commonly used diluent or excipient such as a filler, an extender, a binder, a wetting agent, a disintegrant, a surfactant, and the like. A solid preparation for oral administration may include a tablet, a pill, a powder, granules, a capsule, and the like. Such a solid preparation is prepared by mixing at least one excipient, for example, starch, calcium carbonate, sucrose or lactose, gelatin, and the like with the compound. Also, lubricants such as magnesium stearate, talc, and the like are also used in addition to the simple excipients. A liquid preparation for oral administration may include a suspension, an oral liquid, an emulsion, syrup, and the like. In addition to commonly used simple diluents such as water, liquid paraffin, and the like, the liquid preparation may include various excipients, for example, a wetting agent, a sweetening agent, a fragrance, a preservative, and the like. A preparation for parenteral administration includes a sterile aqueous solution, a non-aqueous solvent, a suspending agent, an emulsifying agent, a freeze-dried preparation, a suppository, and the like. Propylene glycol, polyethylene glycol, a vegetable oil (such as olive oil), an injectable ester (such as ethyl oleate), and the like may be used as a non-aqueous solvent and a suspending agent. Witepsol, Macrogol, Tween 61, cacao butter, laurin butter, glycerogelatin, and the like may be used as a base of the suppository.

The pharmaceutical composition of the present invention may be administered orally or parenterally, and preferably administered parenterally. In the case of parenteral administration, the pharmaceutical composition of the present invention may be administered by intramuscular injection, intravenous injection, subcutaneous injection, intraperitoneal injection, topical administration, transdermal administration, and the like.

A suitable dose of the pharmaceutical composition of the present invention may vary depending on various factors, such as a preparation method, the mode of administration, the age, weight, sex, and pathological condition of a patient, food, an administration time, a route of administration, an excretion rate, and response sensitivity.

The pharmaceutical composition of the present invention may be formulated into a unit dosage form or may be prepared in a large-capacity container by using a pharmaceutically acceptable carrier and/or excipient according to a method that may be easily carried out by a person having ordinary skill in the art to which the present invention pertains. In this case, the formulation may be in the form of a solution in an oily or aqueous medium, a suspension, or an emulsion, or in the form of an extract, a powder, granules, a tablet, or a capsule, and may further include a dispersant or a stabilizing agent.

According to another aspect of the present invention, the present invention provides a method of screening a therapeutic agent for a lung-related disease using the alveolar organoid according to the present invention.

In the present invention, the method of screening a therapeutic agent for a lung-related disease may include:
treating the organoid with a test substance; and
selecting the test substance as a therapeutic agent for a lung-related disease when the expression of a biomarker protein of a lung-related disease or mRNA encoding the same increases or decreases after treatment with the test substance compared to before treatment.

In the present invention, the test substance is a substance expected to prevent, ameliorate, or treat a lung-related disease. For example, the test substance may include low-molecular-weight compounds, antibodies, antisense nucleotides, short interfering RNA, short hairpin RNA, nucleic acids, proteins, peptides, and other extracts, or natural products, but the present invention is not limited thereto.

The terms used in the present invention have been selected as widely used general terms as much as possible in consideration of functions in the present invention, but this may vary according to the intention or precedent of the person skilled in the art, the emergence of new technologies and the like. In addition, in certain cases, there are also terms arbitrarily selected by the applicant, in which case the meaning will be described in detail in the description of the invention. Therefore, the terms used in the present invention should be defined based on the meanings of the terms and the contents throughout the present invention, rather than simply the names of the terms.

Although the terms first, second, etc. may be used to describe various elements, these elements are not limited by these terms. These terms are only used to distinguish one element from another. For example, a first element could be called a second element, and, similarly, a second element could be called a first element, without departing from the scope of the present invention. The term "and/or" includes any and all combinations of one or more of the associated listed items.

Throughout the specification of the present invention, when any certain part is said to "include" any component, this means that it may further include other components, rather than excluding other components unless otherwise stated. In addition, the terms "approximately," "substantially," the like used throughout the specification of the present invention are used at, or in close proximity to, numerical values when manufacturing and material tolerances inherent in the indicated meanings are intended to aid in understanding the present invention. Precise or absolute figures are used to assist in the prevention of unfair use by unscrupulous infringers. The terms "step of" used throughout the specification of the present invention does not refer to "step for."

Throughout the specification of the present invention, the term "combination thereof' included in a Markush type expression refers to a mixture or combination of one or more selected from the group consisting of components disclosed in the Markush type expression, and thus means that the mixture or combination include one or more selected from the group consisting of the components.

### [Mode for Invention]

Hereinafter, preferred embodiments of the present invention are presented in order to aid in understanding the present invention. However, it should be understood that the following embodiments are given by way of illustration only to more easily understand the present invention, and are not intended to limit the present invention.

### [EXAMPLES]

### Example 1: Isolation and culturing of alveolar cells (AT2 cells)

The human lung tissue used in this experiment was obtained during lung surgery and was used under the consent of the patient before surgery. Normal lung tissue was collected, and cells were then isolated after placing and transporting the normal lung tissue on ice in a HBSS solution (Gibco, Gaithersburg, MD) containing an antibiotic-antimycotic solution (Gibco, amphotericin B, penicillin, streptomycin). Specifically, the normal lung tissue excised during surgery for the isolation of human alveolar type 2 cells was washed with an HBSS solution containing an antibiotic-antimycotic solution until no blood was visible. The lung tissue from which blood was removed was cut into small pieces of less than 0.5 mm³ using surgical scissors, put into 20 mL of HBSS containing 0.375 mL of trypsin (10 KU/mL; Sigma), 75 mL of elastase (10 U/mL), and 100 mL of collagenase (type 1 collagenase, 60 mg/mL), and then reacted at a 37°C for 30 minutes in a shaking incubator.

Next, the reacted solution was passed through a 40 mm strainer, collected in a 50 mL Falcon tube, and then centrifuged at 1,500 rpm for 10 minutes at room temperature. The supernatant was discarded, and 3 mL of a Red Blood Cell Lysis buffer (11814389001 Roche) was added, and then reacted at room temperature for 3 minutes. Thereafter, the reaction solution was centrifuged at 1,500 rpm for 3 minutes at room temperature, and the cells were suspended in 10 mL of an SAGM medium (Lonza) (including 10 µM ROCK inhibitor) in the dish, and then placed and cultured at 37°C in a 5% CO₂ incubator.

The SAGM medium was changed once every 2 to 3 days, and when the AT2 cells grew to a confluence of 60% or more, the cells were separated and subcultured using an Animal Component-Free Cell Dissociation kit (Catalog # 05426).

Also, to confirm whether the cultured alveolar cells were AT2 cells, the lamellar body, which is a characteristic marker of AT2 cells, was reacted with Lysotracker fluorescence (green) for 30 minutes, and then observed under a fluorescence microscope.

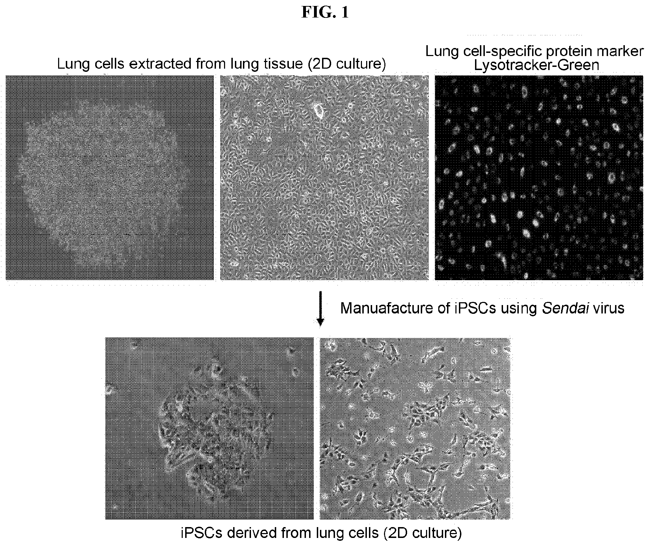

As a result, as shown in FIG. 1, it was confirmed that the cultured cells showed lamellar bodies around the nucleus, indicating that the cultured cells were AT2 cells.

### Example 2: Reprogramming of induced pluripotent stem cells (iPSCs) using Sendai virus

### 2-1. Transduction using Sendai virus

In order to culture alveolar cells in a state suitable for transduction, the human alveolar cells obtained in Example 1 were seeded in the same amount on a 6-well plate, and the number of cells in one well was checked when the alveolar cells had grown to approximately 70 to 80% of at least a 2-well dish.

A Cytotune^{™} 2.0 *Sendai* mix (provided by Invitrogen) was mixed in 1 mL of an SAGM medium at MOI = 5-5-3 (KOS MOI = 5, hc-Myc MOI = 5, hKlf4 MOI = 3), and put into the 6-well plate. After 24 hours, the SAGM medium was exchanged with a new SAGM medium, and the medium was changed every 2 to 3 days.

The alveolar cells were subcultured on day 7 after transduction using the Cytotune^{™} *2.0 Sendai* mix. The timing of the subculturing may be flexible depending on the proliferation rate of the cells, and the cells may be subcultured when the cell density in the plate reaches a confluence of approximately 70 to 80% after the treatment with the *Sendai* mix. The SAGM medium was removed from the wells in which the cells grew, and washed with PBS, and the cells were then detached with TrypLE. The detached cells were collected in an SAGM medium, and then centrifuged at 1,500 rpm for 3 minutes. The supernatant was removed, and the cells were seeded on a cell culture plate prepared in advance. The cell culture plate was coated with vitronectin (provided by Gibco) diluted in PBS at a ratio of 1/100 at room temperature for an hour. Thereafter, the remaining vitronectin was removed, and the cell culture plate was then washed with PBS. The medium was exchanged with an E8 medium within 7 days of cell seeding, and the formation of colonies was observed under a microscope until 3 to 4 weeks while changing the medium every day thereafter.

As a result, it can be seen that the colonies were formed after a predetermined period of time has elapsed. Also, it can be seen that as a predetermined period of time has elapsed further, the iPSC colonies were enlarged in appearance, and round portions of the iPSC colonies were formed in a three-dimensional sense as the iPSC colonies grew to the extent to which the edges of the cells could be distinguished from the existing seeded cells.

### 2-2. Analysis of protein characteristics of induced pluripotent stem cells manufactured using Sendai virus

To check whether the induced pluripotent stem cells (iPSCs) manufactured from the human alveolar cells actually have properties of pluripotent stem cells, the expression of Oct3/4, SSEA-4, and NANOG was confirmed through an immunofluorescence staining method. In a staining process, the cells were first fixed with 4% paraformaldehyde, washed with PBS, and then blocked with a 1% BSA solution. Thereafter, the cells were treated with primary antibodies against Oct3/4, SSEA4, TRA-1-60, TRA-1-81, LIN28, and NANOG (all provided by Santa Cruz Biotechnology), reacted at 4°C for 18 hours, and then washed with PBS. Then, the cells were treated with fluorescent secondary antibodies (Alexa Fluor 594-conjugates, provided by Santa Cruz Biotechnology) against the primary antibodies Oct3/4, SSEA4, TRA-1-60, TRA-1-81, LIN28, and NANOG, and reacted at room temperature for an hour. The cells were washed with PBS, and mounted with a mounting solution, and expression was analyzed using confocal microscopy. 4',6-Diamidino-2-phenylindole (DAPI) was used for the visualization of intracellular nuclei through DNA staining.

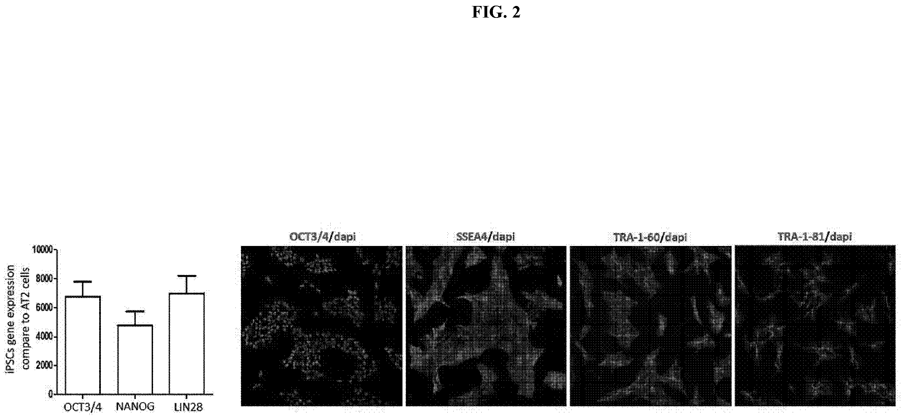

As a result, as shown in FIG. 2, it was confirmed that the iPSC undifferentiated markers Oct3/4, SSEA4, TRA-1-60, TRA-1-81, LIN28, and NANOG were expressed in the induced pluripotent stem cells manufactured from the human alveolar cells.

### Example 3: Subculturing (passaging) and mass culturing of induced pluripotent stem cells (iPSCs) manufactured from human alveolar cells

Among the cells subcultured after transduction in section 2-1 of Example 2, the cells that were not transduced by the virus, that is, were not reprogrammed, were also mixed together. Accordingly, subculturing was performed in the following manner in order to selectively culture only the induced pluripotent stem cells. A new cell culture dish was coated with vitronectin diluted in PBS at room temperature for an hour. Then, the remaining vitronectin was removed, and the cell culture dish was washed with PBS. Colonies were formed when the cells subcultured after transduction in section 2-1 of Example were cultured for approximately 3 to 4 weeks. At this time, the medium was removed from the cells, and the cells were washed with PBS. Then, an E8 medium supplemented with a 10 µM ROCK inhibitor (provided by Sigma Co., Ltd.) was added, and the edges of the induced pluripotent stem cells were then fragmented using a 100 µL pipette tip while being observed under a microscope. An E8 (w/ 10 µM ROCK inhibitor) medium was added to a new cell culture dish, and the fragmented induced pluripotent stem cells were harvested from the E8 medium, placed in a new culture dish, and cultured in an incubator.

As a result, it was confirmed that the colonies of the induced pluripotent stem cells were attached with a small size and an elongated edge shape on day 1, but the size of the colonies also increased with a round shape over time. Also, it was confirmed that the colonies of the induced pluripotent stem cells divided normally when the nucleoli present in the cells were checked.

Next, in order to culture the induced pluripotent stem cells on a large scale, the induced pluripotent stem cells were subcultured, as follows. The fragmented induced pluripotent stem cells were subcultured in an E8 medium to obtain a colony consisting only of induced pluripotent stem cells. Thereafter, the colony was treated with an iPSC-specific cell isolation solution (ReLeSR^{™} provided by STEMCELL Technologies) at room temperature for a minute. After the ReLeSR^{™} solution was removed, the cells were harvested from the E8 medium, and then centrifuged at 1,500 rpm for 5 minutes. The supernatant was discarded, and the pellets were suspended in an E8 (w/ 10 µM ROCK inhibitor) medium. Then, the suspended cells were put into a cell culture dish containing an E8 (w/ 10 µM ROCK inhibitor) medium prepared in advance, and then cultured in an incubator.

As a result, it can be seen that the cell colonies proliferated on day 4 compared to that of day 1. As such, when the ReLeSR^{™} solution was used, the cell colonies were isolated as single cells, which resulted in a slower colony proliferation rate.

### Example 4: Differentiation of induced pluripotent stem cells (iPSCs) into alveolar organoids

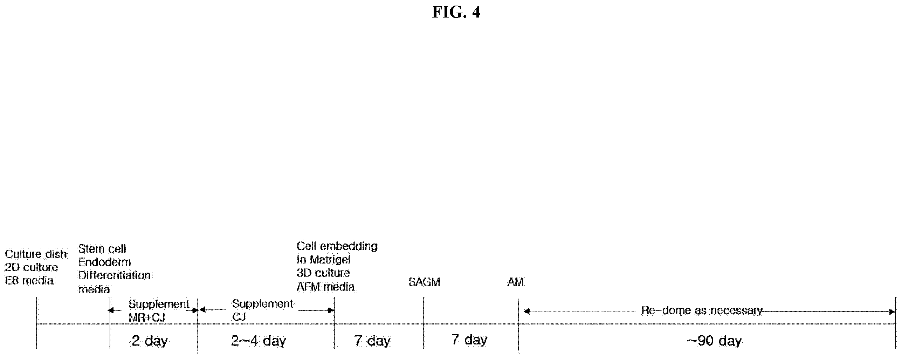

As shown in FIG. 4, alveolar organoids were manufactured by inducing the differentiation of the AT2 cell-derived iPSCs (AT2 iPSCs) obtained in Example 3 into alveolar organoids. This specific method is as follows.

### 4-1. Differentiation of AT2 iPSCs into endoderm cells

The AT2 iPSCs obtained in Example 3 were cultured on a dish coated with vitronectin, and the medium was changed once a day. A TeSR^{™}-E8^{™} (STEMCELL #05990) medium was used as the medium. When the AT2 iPSCs in the dish reached a cell confluence of 70 to 80%, the AT2 iPSCs were cultured using a differentiation medium in a STEMdiff^{™} Definitive Endoderm kit (STEMCELL #05110). MR and CJ supplements were added to the medium during culturing, and cultured for 2 days, and the CJ supplement was then added alone and cultured for 2 to 4 days according to the manufacturer's instructions.

To check whether the AT2 iPSCs were differentiated into endoderm cells, the expression of the endoderm-specific factors SOX17 and FOXA2 was checked using the immunofluorescence staining method described in section 2-2 of Example 2.

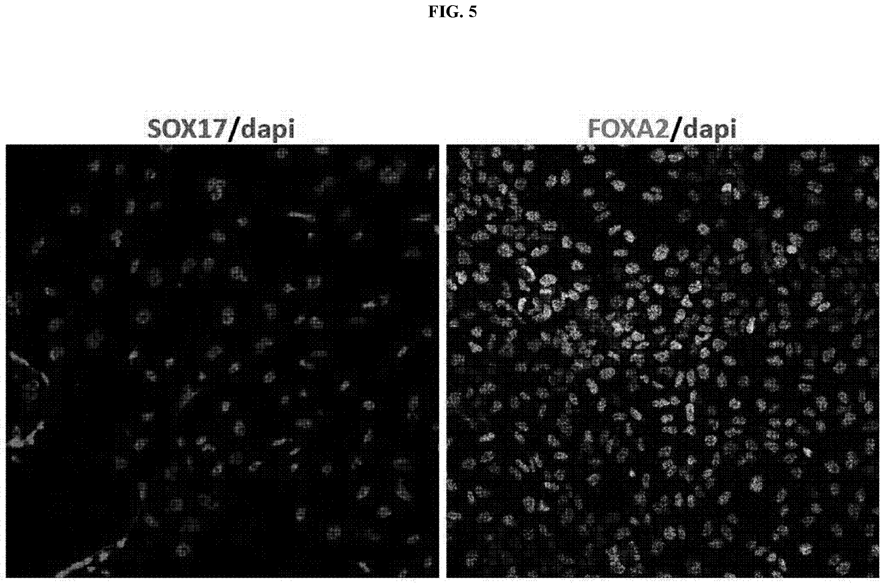

As a result, as shown in FIG. 5, it can be seen that the endoderm-specific factors SOX17 and FOXA2 were expressed.

### 4-2. Differentiation of endoderm cells into alveolar organoids

In section 4-1 of Example 4, the differentiation of AT2 iPSCs into endoderm cells was carried out in a culture dish using a 2D culture method, and the subsequent processes were performed in a 3D dome-shaped culture method using Matrigel (Corning #356231). In this case, 60% Matrigel was used.

To convert the endoderm cells of section 4-1 of Example 4 into anterior foregut endoderm cells, which are precursors of lung and airway cells, the endoderm cells were cultured for 7 days using an anterior foregut endoderm medium (AFM). Thereafter, in order to induce the differentiation of the endoderm cells into an alveolar progenitor, the endoderm cells were cultured for 7 days using an SAGM^{™} (Lonza #CC-3118) medium. After the culturing in the SAGM medium was completed, the alveolar progenitor was subcultured in an alveolar medium (AM) to induce the maturation of alveolar organoids. When the cells continued to divide during culturing in the AM so that the cell density of the spheroid became too high, the spheroid was isolated using Accutase (Corning #25-058-CI). Then, a re-dome process of embedding the spheroid in Matrigel was repeatedly performed. Then, the spheroid was cultured for up to 90 days. Meanwhile, when the spheroid maintained a spherical shape and an appropriate cell density, and the lumen was observed, the spheroid was continuously cultured in the AM without a re-dome process. It was confirmed through fluorescence staining whether the cells constituting the spheroid included alveolar epithelial cells. The specific components of the used AFM and AM are shown in Tables 1 and 2, respectively.

**[Table 1]**

| **AFM(Anterior Foregut endoderm Media) component** | |
|---|---|
| Ham's F12(Gibco^{™} #11765054) | 500 mL |
| Dexamethasone (Sigma-Aldrich #D4902) | 50 nM |
| 3-Isobutyl-1-methylxanthine(IBMX) (Sigma-Aldrich #I5879) | 100 µM |
| B27 supplement (Gibco^{™} #17504004) | 2% |
| N2 supplement (Gibco^{™} #17502048) | 1% |
| 7.5% Bovine serum albumin Fraction v (Gibco^{™} 15260037) | 1% |
| HEPES (Gibco^{™} #15630080) | 15 mM |
| CaCl₂ (Sigma-Aldrich #21115) | 0.8 mM |
| 8-BrcAMP (Sigma-Aldrich #B7880) | 100 µM |
| Recombinant Human KGF (PEPROTECH #100-19) | 10 ng/ml |
| Recombinant Human FGF-10 (PEPROTECH #100-26) | 10 ng/ml |
| Anti Antibiotic-Antimycotic(Gibco^{™} #15240096) | 1% |
| Ascobic acid (Sigma-Aldrich #A4544) | 50 µg/ml |
| Glutamax (Gibco^{™} #35050061) | 2 mM |
| Recombinant Human BMP-4 (rndsystems #314-BP) | 10 ng/ml |
| CHIR99021 (TOCRIS #4423) | 3 µM |

**[Table 2]**

| **AM(Alveolar Media) component** | |
|---|---|
| Ham's F12(Gibco^{™} #11765054) | 500 mL |
| Dexamethasone (Sigma-Aldrich #D4902) | 50 nM |
| 3-Isobutyl-1-methylxanthine(IBMX) (Sigma-Aldrich #I5879) | 100 µM |
| B27 supplement (Gibco^{™} #17504004) | 2% |
| N2 supplement (Gibco^{™} #17502048) | 1% |
| 7.5% Bovine serum albumin Fraction v (Gibco^{™} 15260037) | 1% |
| HEPES (Gibco^{™} #15630080) | 15 mM |
| CaCl₂ (Sigma-Aldrich #21115) | 0.8 mM |
| 8-BrcAMP (Sigma-Aldrich #B7880) | 100 µM |
| Recombinant Human KGF (PEPROTECH #100-19) | 10 ng/ml |
| Recombinant Human FGF-10 (PEPROTECH #100-26) | 10 ng/ml |
| Anti Antibiotic-Antimycotic(Gibco^{™} #15240096) | 1% |

### 4-3. Identification of differentiation characteristics of alveolar organoids

Immunofluorescent staining was performed to confirm the characteristics of alveolar organoids according to progression of a differentiation process.

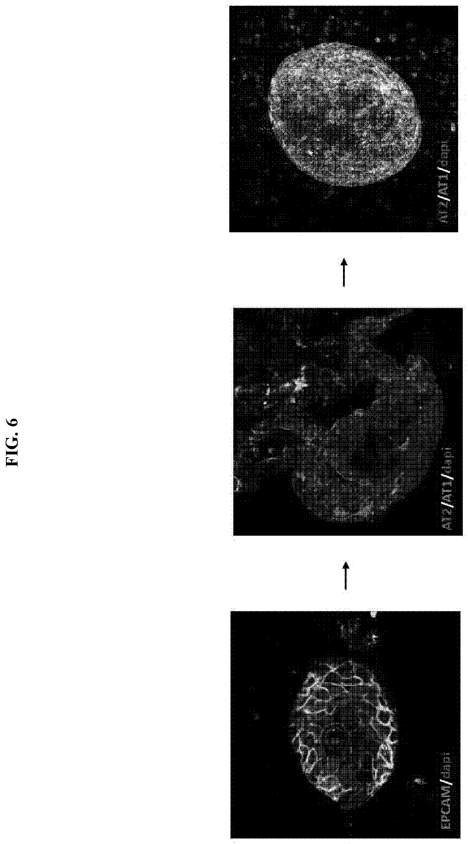

As a result, as shown in FIG. 6, it can be seen that EPCAM serving as an epithelial cell factor was expressed at the beginning of differentiation, and the expression of alveolar type 2 cells (AT2) and alveolar type 1 cells (AT1) was observed as differentiation progressed. Therefore, it can be seen that alveolar organoids containing AT2 cells and AT1 cells were formed inside the organoids when differentiation progressed further.

The above description of the present invention has been given by way of illustration only. Therefore, those skilled in the art to which the present invention pertains will appreciate that the present invention can be embodied in other specific forms without changing the technical spirit or essential features of the present invention. Therefore, it should be understood that the embodiments described above are for the purpose of illustration only, and not intended to be limiting in all respects.

### [Industrial Applicability]

The alveolar organoid manufactured according to the present invention is differentiated from human alveolar cell-derived induced pluripotent stem cells, and thus induced pluripotent stem cells may repeatedly and efficiently differentiate into alveolar organoids and actually reproduced in a similar manner to alveoli due to the epigenetic memory of alveolar cells. Therefore, the present invention is industrially applicable because the alveolar organoids are expected to be effectively used for research on the pathological process of respiratory diseases, screening for finding therapeutic drugs, and the like.

## Claims

1. A method of manufacturing an alveolar organoid from alveolar cell-derived induced pluripotent stem cells, comprising:
a) culturing alveolar cell-derived induced pluripotent stem cells in a medium for inducing endoderm cell differentiation to induce the differentiation of the induced pluripotent stem cells into endoderm cells;
b) inducing the differentiation of the endoderm cells of step a) into anterior foregut endoderm cells in an anterior foregut endoderm medium (AFM);
c) inducing the differentiation of the anterior foregut endoderm cells of step b) into an alveolar progenitor in a progenitor differentiation medium; and
d) culturing the alveolar progenitor of step c) in an alveolar medium (AM).

2. The method of claim 1, wherein the alveolar cells are alveolar type 2 cells.

3. The method of claim 1, wherein the AFM comprises one or more selected from the group consisting of Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, antibiotic-antimycotic, ascorbic acid, Glutamax, recombinant human BMP-4, and CHIR99021.

4. The method of claim 1, wherein the AM comprises one or more selected from the group consisting of Ham's F12, dexamethasone, 3-isobutyl-methylxanthine (IBMX), B27 supplement, N2 supplement, bovine serum albumin (BSA), hydroxyethyl piperazine ethane sulfonic acid (HEPES), calcium chloride (CaCl₂), 8-Br-cAMP, recombinant human KGF, recombinant human FGF-10, and antibiotic-antimycotic.

5. The method of claim 1, wherein steps b) to d) are performed by a three-dimensional (3D) culture method.

6. An alveolar organoid manufactured using the method of any of claims 1 to 6.

7. A kit for manufacturing an alveolar organoid, comprising a medium for inducing endoderm cell differentiation, an anterior foregut endoderm medium (AFM), a progenitor differentiation medium, and an alveolar medium (AM).

8. The kit of claim 7, further comprising instructions describing the method of manufacturing an alveolar organoid from the alveolar cell-derived induced pluripotent stem cells as defined in claim 1.
